(19) **Europäisches Patentamt**

**European Patent Office**

**Office européen des brevets**

(11) **EP 1 489 411 A1**

(12) **EUROPEAN PATENT APPLICATION**
published in accordance with Art. 158(3) EPC

(43) Date of publication:
**22.12.2004 Bulletin 2004/52**

(51) Int Cl.7: **G01N 30/88**, G01N 30/72,
G01N 30/04, G01N 27/62

(21) Application number: **03712851.9**

(22) Date of filing: **24.03.2003**

(86) International application number:
**PCT/JP2003/003521**

(87) International publication number:
**WO 2003/081229 (02.10.2003 Gazette 2003/40)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **25.03.2002 JP 2002082821**

(71) Applicant: **TEIJIN LIMITED**
**Osaka-shi, Osaka 541-0054 (JP)**

(72) Inventors:
- **SAKAI, M.; c/o Teijin Limited**
  **Hino-shi, Tokyo 191-0065 (JP)**
- **SUGIMOTO, Y.; c/o Teijin Limited**
  **Hino-shi, Tokyo 191-0065 (JP)**

(74) Representative: **Hallybone, Huw George et al**
**Carpmaels and Ransford,**
**43 Bloomsbury Square**
**London WC1A 2RA (GB)**

(54) **METHOD OF ASSAYING COENZYMES A IN BIOLOGICAL SAMPLE**

(57) A method for assaying concentrations of Coenzyme A (CoA) molecules in biological samples with high sensitivity and high reproducibility, the method comprising a step of extraction from a biological sample using a strongly acidic solution, a step of solid phase extraction, a step of adding an internal standard substance, and a step of detection by LC-MS.

**Fig. 1**

EP 1 489 411 A1

**Description**

FIELD OF THE INVENTION

The present invention relates to a method for quantitative assay of Coenzyme A (hereinafter referred to as "CoA") molecules in biological samples.

BACKGROUND ART

CoA molecules are important class of compounds indispensable for maintaining life functions, being involved in the paths of biosynthesis, decomposition and conversion of fatty acids, hormone synthesis and regulation, the TCA cycle, etc. Methods for accurately assaying CoA molecules concentrations in biological samples have been a target of research, with emphasis on the role of such concentrations as markers for CoA molecules in functional analysis research on lipid metabolism.

One of the CoA molecules, malonyl CoA, is a constituent of the lipid metabolism mechanism involved in fatty acid oxidation in the mitochondria and lipid synthesis, and therefore performs a crucial role for regulation of lipid metabolism; it has received much attention as a regulatory factor essential for regulating lipid energy metabolism in the heart and skeletal muscle and expression of neuropeptide Y in the cerebral hypothalamus, as well as for controlling dietary intake and energy consumption.

Various methods for assaying CoA molecules have been developed in the past, including enzyme methods and HPLC. Examples of enzymatic assay methods include the method of Guynn et al. (Guynn, R.W., Methods Enzymol., 1975, 35, 312) and the method of McGarry et al. (McGarry, J.D., J. Biol. Chem., 1978, 253, 22, 8291). For example, when the assay target is malonyl CoA, malonyl CoA-dependent uptake of radiolabeled acetyl CoA or the like is measured. However, this method has limited applicability because its assay target is a specific CoA and adjustment must be made due to the copresence of other CoA molecules during the assay, leading to inaccurate measured values in some cases, while the procedure is also complicated.

Numerous reports have been published on assay of CoA molecules by UV-HPLC (see, for example, Hosokawa, Y., Anal. Biochem., 1978, 91, 1, 370, Demoz, A., J. Chromatogr., B: Biomed. Appl., 1995, 667, 1, 148). CoA concentrations in muscle, cardiac and hepatic biological samples are assayed by this method. However, due to the low sensitivity of UV-HPLC, it has poor reproducibility for highly contaminated samples such as those from organs, with brain concentrations being particularly difficult to measure, and therefore a method offering higher sensitivity and reproducibility has been desired.

LC-MS has been developed as an alternative method for achieving higher sensitivity (see Buchholz, A., Anal. Biochem., 2001, 295, 129). This method accomplishes assay of acetyl CoA concentrations in cells using a three-dimensional ion trap mass spectrometer. Although success has been achieved with this method for detection and peak separation of a particular CoA as the assay target, the accuracy and reproducibility are insufficient because the method is an absolute calibration curve method and does not use an internal standard substance. Still, the assay target is acetyl CoA which has low polarity and is easily separated by HPLC. Its additional advantages are that cellular samples have low amounts of contaminants that can interfere with measurement, and that acetyl CoA concentrations are high in samples. The method cannot be adequately applied, however, for assay of CoA molecules in cases of highly polar forms whose separation is relatively difficult by HPLC, in cases of samples such as animal organs with high amounts of contaminants that can interfere with measurement, and in cases of low concentrations in samples (such as malonyl CoA in animal organs).

The present invention solves the problems described above by providing a concentration assay method with excellent sensitivity and reproducibility for CoA molecules in biological samples.

DISCLOSURE OF THE INVENTION

As a result of much diligent research on the subject described above, the present inventors have discovered the following method. Specifically, the invention provides a method for assaying concentrations of Coenzyme A molecules in biological samples, the method being characterized by comprising a step of extraction from a biological sample using a strongly acidic solution, a step of solid phase extraction, a step of adding an internal standard substance, and a step of detection by LC-MS.

The invention further provides the aforementioned method for assaying Coenzyme A molecules characterized in that the step of extracting the Coenzyme A molecule from the biological sample is a step wherein a freeze-shattered biological sample is agitated in a perchloric acid solution and the supernatant is subjected to centrifugal separation.

The solid phase extraction step is characterized by being a step wherein the supernatant obtained by extraction of the Coenzyme A with a strongly acidic solution is neutralized, and then applied to a reverse phase cartridge packed with silica gel containing an octadecylsilyl group or octylsilyl group, washed with an aqueous solvent, and eluted with an organic solvent. In particular, the supernatant is applied after conditioning the reverse phase cartridge with acetonitrile and 1 M ammonium acetate solution, and elution is performed with an acetonitrile and ammonium acetate mixture.

The invention still further provides the aforementioned method for assaying Coenzyme A molecules characterized in that the Coenzyme A molecule is a fatty acid Coenzyme A ester, and the internal standard substance is a structural analog of the Coenzyme A molecule.

The invention still further provides the aforementioned method for assaying Coenzyme A molecules characterized in that the fatty acid Coenzyme A ester is a Coenzyme A ester of a short chain fatty acid with 2-8 carbons in the main carbon chain, the structural analog has a difference of no more than 3 carbons with the Coenzyme A molecule and has at least 3 of the hydrogens of the main chain substituted with deuterium, or is substituted with $^{13}$C, and particularly in that the Coenzyme A molecule is malonyl CoA and the structural analog is acetyl CoA-$d_3$, methylmalonyl CoA-$d_3$, methylmalonyl CoA-$d_4$, propionyl CoA-$d_3$, propionyl CoA-$d_5$ or malonyl CoA-$^{13}C_3$.

## BRIEF DESCRIPTION OF THE DRAWINGS

Fig. 1 is a chromatogram for assay using a muscle sample from a Wistar rat.
Fig. 2 is a chromatogram for assay using a liver sample from a Wistar rat.
Fig. 3 is a chromatogram for assay using a brain sample from a Wistar rat.

## BEST MODE FOR CARRYING OUT THE INVENTION

The invention relates to a method for assaying CoA molecules, which comprises extraction from a biological sample with a strongly acidic solution, followed by concentration if necessary, addition of an internal standard substance, preparation of an HPLC injection sample, HPLC separation of the CoA molecule and the internal standard substance, detection of the CoA molecule and the internal standard substance with a mass spectrometer, and quantitation from the area ratio of the detected CoA to be assayed and the internal standard substance.

Extraction of the CoA molecule from a biological sample with a strongly acidic solution may be performed from any biological sample containing a CoA molecule, and as specific examples there may be mentioned human and animal organs, human, animal and plant tissues or cells, microbes, and the like. Assay of CoA molecules in organs such as muscle, liver and brain is particularly useful.

As CoA molecules to be assayed there may be mentioned acyl CoA molecules having acylated thiol groups, oxidized CoA molecules having oxidatively bonded thiol groups, and N-acyl CoA molecules having the primary amine acylated. Although some of the CoA molecules mentioned here are not found in biological samples, they are used for the purpose of research such as functional analysis and are therefore important for evaluating effects in drug development.

As specific examples of acyl CoA molecules there may be mentioned acetoacetyl CoA, malonyl CoA, succinyl CoA, 3-hydroxy-3-methylglutaryl CoA, glutaryl CoA, CoA, acetyl CoA, benzoyl CoA, phenylacetyl CoA, isobutyryl CoA, isovaleryl CoA, butyryl CoA, betamethylcrotonyl CoA, tiglyl CoA, 3-hydroxypropionyl CoA, crotonyl CoA, hexanoyl CoA, methylmalonyl CoA, propionyl CoA, acryloyl CoA, arachidonyl CoA, decanoyl CoA, elaidoyl CoA, oleoyl CoA, palmitoleoyl CoA, palmitoyl CoA, linoleoyl CoA, lauroyl CoA, myristoleoyl CoA, nervonoyl CoA, stearoyl CoA, octanoyl CoA, myristoyl CoA, arachidonyl CoA, heptadecanoyl CoA, nonadecanoyl CoA, docosahexanoyl CoA, pentadecanoyl CoA, betahydroxybutyryl CoA, heptanoyl CoA, valeryl CoA, 2-butenoyl CoA and stearoyl CoA.

As specific examples of N-acyl CoA molecules there may be mentioned N-butyryl CoA, N-decanoyl CoA and N-hexanoyl CoA.

As specific examples of oxidized CoA molecules having oxidatively bonded thiol groups there may be mentioned oxidized CoA and CoA glutathione disulfide.

Among these are preferred acetyl CoA, CoA, succinyl CoA, acetoacetyl CoA, 3-hydroxy-3-methylglutaryl CoA, propionyl CoA, methylmalonyl CoA, malonyl CoA, 3-hydroxypropionyl CoA, acryloyl CoA, oleoyl CoA, stearoyl CoA, linolenyl CoA, arachidonyl CoA, palmitoyl CoA, stearoyl CoA, isobutyryl CoA, oxidized CoA and CoA glutathione disulfide, with short chain ($\leq C_8$) fatty acid CoA esters and thioesters such as acetyl CoA, CoA, succinyl CoA, acetoacetyl CoA, 3-hydroxy-3-methylglutaryl CoA, propionyl CoA, methylmalonyl CoA, malonyl CoA, isobutyryl CoA and CoA glutathione disulfide being particularly suitable for assay.

The strongly acidic solution used for extraction of the CoA molecule from the biological sample will generally be a solution which accomplishes protein denaturation of the biological sample and extracts the compounds to be assayed, and as specific preferred examples there may be mentioned trichloroacetic acid solution and perchloric acid solution. There are no particular restrictions on the specific extraction method used, and it may be appropriately selected depending on the sample to be measured and the assay target. For example, after freezing a biological tissue such as muscle or brain with liquid nitrogen and then pulverizing it, a strongly acidic solution may be added to the prescribed concentration and the mixture adequately stirred and centrifugally separated, and then the supernatant used as the biological sample extract.

Preferred examples of internal standard substances to be added include the aforementioned CoA molecules or their isotopes. Based on the nature of the internal standard method, the CoA to be assayed and the internal standard substance preferably have a relationship such that their physical and chemical properties are similar and both exhibit the same behavior during the method procedure, but can be separated during detection without mutual interference. In order to satisfy this relationship, it is preferred to select a CoA analog or CoA isotope which satisfies the selection criteria described below for the internal standard substance.

A CoA analog has a difference of no more than 3 carbons compared to the CoA to be assayed, and when the main carbon chain of the CoA to be assayed has a functional group such as alcohol, amine, carboxylic acid or the like, the internal standard substance also preferably has the same functional group. A CoA isotope has at least 3 of the hydrogens of the main chain of the acyl group of the CoA to be assayed substituted with deuterium, or is substituted with $^{13}$C, and it preferably satisfies the aforementioned criteria for a CoA analog.

Because some CoA molecules are essentially biologically derived, the internal standard substance used is preferably artificially synthesized, such as the above-mentioned deuterium or $^{13}$C form, or N-acyl CoA or the like. For assay of rat liver malonyl CoA, for example, acetyl CoA cannot be used as the internal standard substance because of the high endogenous concentration of acetyl CoA, and therefore acetyl CoA-$d_3$, acetoacetyl CoA-$d_5$, methylmalonyl CoA-$d_3$, methylmalonyl CoA-$d_4$, propionyl CoA-$d_3$, propionyl CoA-$d_5$, isobutyryl CoA-$d_7$ or methylmalonyl CoA-$^{13}$C$_3$ is preferably used.

Addition of the internal standard substance may be either addition to the supernatant obtained by CoA extraction from the biological sample with the strongly acidic solution, or initial addition to the strongly acidic solution. If the behavior of the internal standard substance during the treatment process is too dissimilar, it may be added during HPLC sample preparation, or it may perform only a calibrating role for ionization of the mass spectrometer.

Since the extraction supernatant will be a strongly acidic solution, it is adjusted to a pH in the neutral range by neutralization, solvent exchange or the like before being supplied to HPLC. The HPLC-injected sample is adjusted to a pH of 3-10. The assay is most preferably carried out in the neutral pH range of 3-8 from the standpoint of sensitivity, reproducibility and stability. At the acidic end of pH 2 and below, chromatographic peak tailing may occur due to the phosphate groups of the CoA, or adsorption to the column may become significant. At the basic end of pH 10 and above, decomposition of the CoAs may become significant. The reagent used for adjustment of the pH may be a pH regulator having a buffer effect, such as ammonium acetate or potassium phosphate.

For assay using a biological sample with low CoA concentration, such as brain or the like, or for highly sensitive analysis, the sample is concentrated. As preferred methods of concentration there may be mentioned lyophilization, concentration under reduced pressure, liquid-liquid extraction, solid phase extraction, online concentration and the like, among which solid phase extraction is preferred.

Solid phase extraction may be accomplished using a commercially available reverse phase, normal phase or ion-exchange cartridge, but a reverse phase cartridge has excellent reproducibility and is therefore most suitable for assay of CoA molecules. A normal phase system is poorly suited because of the high polarity of CoAs, while with an ion-exchange system, the salt concentration will be too high when extraction is performed with the strongly acidic solution, and therefore retention on the carrier will be difficult.

The solid phase extraction procedure involves first conditioning the solid phase cartridge using an organic solvent, aqueous solvent or the like, neutralizing the supernatant obtained by extraction of the CoA with the strongly acidic solution and applying it to the cartridge for adsorption of the CoA to the solid phase cartridge, and then washing with an aqueous solvent and subsequently eluting the adsorbed CoA.

The conditioning is carried out with an organic solvent and a high-concentration salt solution. When a reverse phase cartridge is used, the conditioning is carried out with acetonitrile and 1 M ammonium acetate. Specifically, 100% acetonitrile is injected into the solid phase cartridge, and then, in order to avoid precipitation of the salt, the acetonitrile concentration of the cartridge is reduced using 50% aqueous acetonitrile, after which 1 M aqueous ammonium acetate is added for conditioning of the cartridge.

With a low ammonium acetate concentration of about 50 mM, it becomes impossible to completely retain the CoA in the reverse phase cartridge, thereby impairing the yield.

The organic solvent used for conditioning may be acetonitrile, or another commonly used solvent such as methanol or 2-propanol. As salt solutions to be used there may be mentioned ammonium acetate, or ammonium formate, ammonium carbonate, sodium phosphate, potassium phosphate or the like. The concentration is preferably 200 mM to 2 M.

The aqueous solution used for washing is preferably water, but it may also contain a salt such as ammonium acetate or sodium phosphate. For a further increased washing effect, an organic solvent such as acetonitrile or methanol may also be included in an amount which does not elute the CoA. The proportion of water in the CoA washing solvent is at least 50%, and an aqueous solution containing no organic solvent is particularly preferred for assay of short-chain fatty acid CoA esters because of their high polarity.

Next, the eluting solvent is used to elute the assay target, CoA, retained in the cartridge. The elution solvent used may be a mixture of an organic solvent commonly used for solid phase extraction, such as methanol, acetonitrile, 2-propanol, acetone or tetrahydrofuran, with water or the aqueous solvent used as the washing solution mentioned above, and the mixing ratio (0-100%) may be appropriately determined based on the elution kinetics of the CoA assay target and internal standard substance, as well as the elution kinetics of the contaminants.

In the case of a short-chain fatty acid CoA ester such as malonyl CoA, elution may be performed with an approximately 20% organic solvent solution, but the concentration is preferably 20-75% in consideration of subsequent distilling off of the solvent. At close to 100%, elution of contaminants becomes a significant factor. The solvent of the eluate is distilled off and the residue is redissolved with a redissolving solution. If no internal standard substance has been added to the extract obtained with the strongly acidic solution, a prescribed amount thereof may be added at this point.

For HPLC separation of the CoA and the internal standard substance, a liquid chromatography separation column which is commercially available for ordinary reverse phase chromatography may be used, but a silica-based filler having bonded octadecylsilane groups (C18) is preferred. There may also be used silica-based fillers having bonded octylsilane groups (C8) or amide-based, and particularly carbamoyl chemical bond-type silica gels.

The column size may be any commercially available size for analysis, and the assay may be carried out with an inner diameter of 1 mm to 10 mm and a length of 50 mm to 250 mm. However, there is no limitation to this size range, and basically it is sufficient if the assay target substance is properly retained and clear peaks are shown. The mobile phase used may be any solvent ordinarily used for reverse phase HPLC, and it preferably contains a volatile or sublimating salt such as ammonium acetate.

As specific examples of suitable mass spectrometers for detection of the CoA and internal standard substance by mass spectrometry there may be mentioned quadrupole spectrometers, sector spectrometers, triple quadrupole spectrometers, ion-trap spectrometers, time-of-flight spectrometers, quadrupole time-of-flight hybrid spectrometers, and the like. Among these, quadrupole spectrometers and triple quadrupole spectrometers are preferred, with triple quadrupole spectrometers being most preferred. The detection conditions will include setting of the mass unit for the compound and detection of the peaks separated by the analysis column.

For quantitation from the area ratio of the detected CoA assay target and the internal standard substance, a sample prepared for the calibration curve is assayed under the detection conditions described above, and the peak area ratios and concentrations of the CoA assay target and the internal standard substance are used to create the calibration curve. The calibration curve is created from a first-order regression line by the least square method, and the peak area ratios of the CoA assay target and the internal standard substance obtained by actual sample measurement are used for inverse regression, to calculate the concentration of the CoA assay target.

EXAMPLES

Examples of the CoA assay method of the invention, for assay of malonyl CoA in muscle, liver and brain, will now be explained.

1. Preparation of standard solutions

A few milligrams of a lithium salt of malonyl CoA was accurately measured out with a precision scale and then dissolved with 6% perchloric acid to a concentration of 10 mM to prepare a standard stock solution. The standard stock solution was gradually diluted with 6% perchloric acid to prepare 10 $\mu$M, 5 $\mu$M, 1 $\mu$M, 500 nM, 100 nM, 50 nM and 10 nM standard solutions for the calibration curve. Similarly, the stock solution was gradually diluted with 6% perchloric acid to prepare 50 $\mu$M, 5 $\mu$M and 500 nM standard solutions for confirmation of reproducibility (QC).

2. Preparation of internal standard solution (IS)

A few milligrams of acetyl CoA-$d_3$ was accurately measured out with a precision scale and then dissolved with 6% perchloric acid to a concentration of 10 mM to prepare a standard stock solution. The standard stock solution was diluted with 6% perchloric acid to prepare a 5 $\mu$M standard solution for the calibration curve. The acetyl CoA-$d_3$ was synthesized according to the method of Simon (Simon, E.J., J.A.C.S., 1953, 75, 2520) using CoA and acetic anhydride-$d_6$ as the starting substances.

3. Preparation of sample solution for assay

1) Preparation of biological sample extract

Immediately after extracting muscle, liver or brain tissue from Wistar rats, it was frozen in liquid nitrogen and pulverized, after which 6% perchloric acid was added to 5 ml/g wet wt. and the mixture was thoroughly stirred. Centrifugation was performed at 9000 rpm and the supernatant was used as the biological sample extract.

2) Sample pretreatment

For assay in muscle and liver: To 300 μL of a 1 M ammonium acetate solution (unregulated pH) there was added 200 μL of the calibration curve standard solution, biological sample extract or, for QC, a pooled biological extract solution from multiple individuals, 20 μL of IS solution, and only for QC, 20 μL of QC standard solution (for QC), to prepare solid phase extraction samples.

For assay in brain: To 450 μL of a 1 M ammonium acetate solution (unregulated pH) there was added 400 μL of the calibration curve standard solution, biological sample extract or, for QC, a pooled biological extract solution from multiple individuals, 20 μL of IS solution, and only for QC, 20 μL of QC standard solution (for QC), to prepare solid phase extraction samples.

After conditioning 100 mg/l mL of BondElute C18 as a solid phase extraction cartridge (product of Varian) with 1 mL of acetonitrile, 1 mL of 50% aqueous acetonitrile and 1 mL of 1 M ammonium acetate solution (unregulated pH), the solid phase extract sample was applied. It was then washed with 1 mL of water, after which the solution eluted with 1 mL of a mixture of acetonitrile/50 mM ammonium acetate solution (unregulated pH) = 2/8 was lyophilized and the solvent was distilled off. Also, 200 μL of water was added to the residue for dissolution to form an assay sample solution, and 20 μL thereof was used in an LC/MS/MS system.

4. Measuring apparatuses and conditions

1) Measuring apparatuses

The following apparatuses and devices were used as an LC/MS/MS system.

| | |
|---|---|
| HPLC system | Agilent 1100 (Agilent) |
| Autosampler | Shimadzu SIL-HTc (Shimadzu) |
| Mass analyzer | API3000 (Applied Biosystems) |

2) HPLC conditions

The HPLC analysis conditions were AQUA C18 as the analysis column and an AQUA C18 guard column as the guard column, with a column temperature of 25°C, and a 10 mM aqueous ammonium acetate (unregulated pH) solution (Solution A) and methanol (Solution B) as the mobile phase, at a flow rate of 1 ml/min, under the gradient conditions shown in Table 1.

Table 1

| HPLC gradient conditions | | |
|---|---|---|
| Time (min) | Solution A (%) | Solution B (%) |
| 0 | 97 | 3 |
| 0.5 | 97 | 3 |
| 3.5 | 70 | 30 |
| 4.0 | 70 | 30 |
| 4.5 | 97 | 3 |
| 6.5 | END | |

3) MS conditions

Mass analysis was conducted by the ionization method (turbo ion spray, positive mode), for measurement of malonyl CoA ion (Q1: 854(m/z), Q3: 347(m/z)) and acetyl CoA-$d_3$ ion (Q1: 813(m/z), Q3: 306(m/z)).

4) Calculation of quantitative values

Peak identification was carried out based on the retention time obtained by MRM (malonyl CoA: m/z 854→347, IS: m/z 831→306) and the mass number of the monitor ion, and quantitation was conducted by the internal standard method based on the peak area ratio for malonyl CoA and IS. The calibration curve formula and the quantitative values for the QC sample and biological sample extracts were determined as follows.
Calibration curve formula: Determined by the least square method (weighting: 1/x), using the relationship between the malonyl CoA peak area ratio (y) and concentration (x) with respect to IS.

$$y = ax + b$$

x: concentration, y: peak area ratio
QC sample and biological sample extract concentrations: Determined by the following formula according to the calibration curve method.

$$x = (y-b)/a$$

x: Measured concentration .

5. Results

1) Linearity: The calibration curve sample was measured according to the analysis method described above, and a calibration curve was created (Table 2). A weighting of 1/X was used, and the linearity was satisfactory with a correlation coefficient (r) of 0.9998 and a relative error (RE) of -7.5 to +6.8%.

Table 2

| Calibration curve data | | |
|---|---|---|
| Theoretical (nM) | Found (nM) | Relative error (%) |
| 10 | 10.7 | 6.8 |
| 50 | 46.3 | -7.5 |
| 100 | 94.8 | -5.2 |
| 500 | 506 | 1.2 |
| 1,000 | 1,040 | 4.4 |
| 5,000 | 5,070 | 1.4 |
| 10,000 | 9,890 | -1.1 |
| Correlation coefficient (r) | 0.9998 | |

2) Reproducibility
As samples for confirmation of reproducibility (QC), the malonyl CoA concentrations were assayed using a blank sample (Q0) and QC samples containing 500 nM, 5 µM and 50 µM QC standard solutions (Q1, Q2, Q3, N=3 for each concentration). QC1 to QC3 represent the obtained quantitative values minus Q0, and as shown in Tables 3 to 5, all of the values were satisfactory, with a purity (%CV) of 1.7-9.1% and a trueness (%RE) of -4.3 to 5.7% for the muscle concentration, a purity (%CV) of 3.0-8.2% and a trueness (%RE) of -5.6 to 9.1% for the liver concentration, and a purity (%CV) of 2.0-7.5% and a trueness (%RE) of -3.6-6.9% for the brain concentration.

3) Assay of malonyl CoA concentrations in biological samples

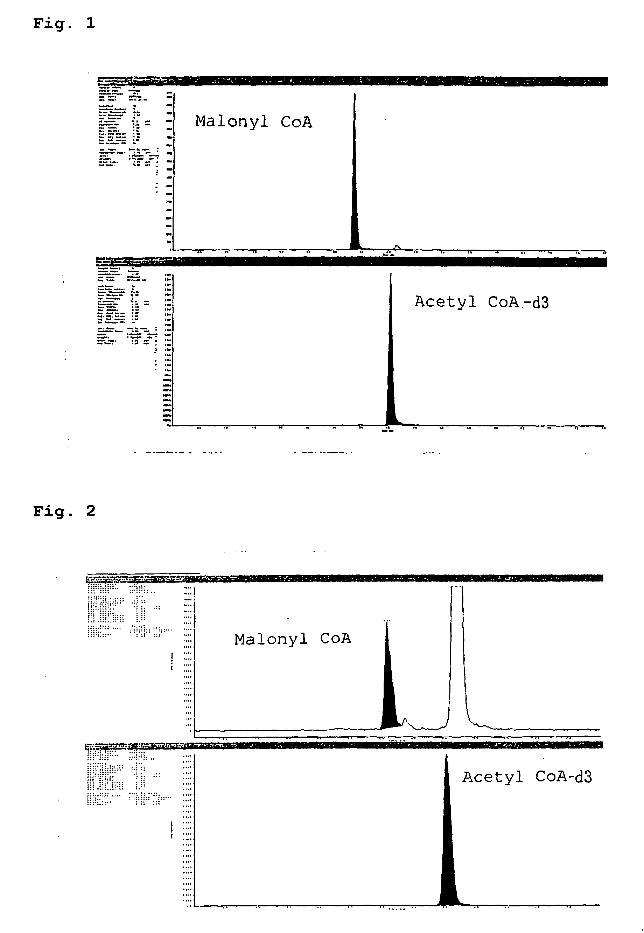

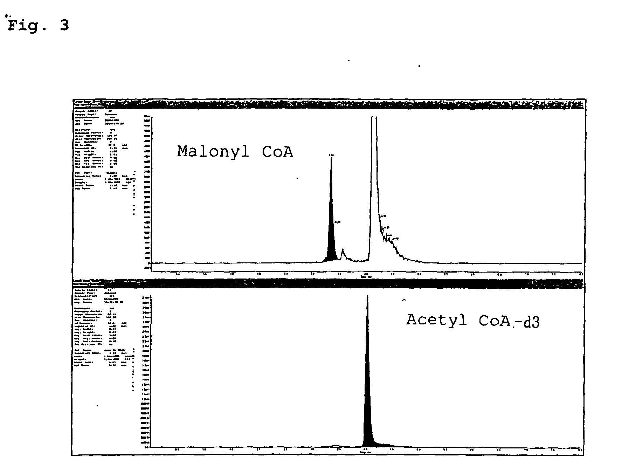

Malonyl CoA concentrations were assayed in the muscle, liver and brain of six Wistar rats. The chromatograms are shown in Fig. 1 and Fig. 3, and the measurement results are shown in Table 6 and Table 8.

Table 6

| Malonyl CoA concentrations in Wistar rat muscle | |
| --- | --- |
| Wistar rat individual No. | Malonyl CoA concentration (nmol/g wet wt.) |
| 1 | 5.94 |
| 2 | 5.21 |
| 3 | 6.41 |
| 4 | 6.01 |
| 5 | 4.54 |
| 6 | 4.12 |

Table 7

| Malonyl CoA concentrations in Wistar rat liver | |
| --- | --- |
| Wistar rat individual No. | Malonyl CoA concentration (nmol/g wet wt.) |
| 1 | 0.42 |
| 2 | 0.67 |
| 3 | 0.44 |
| 4 | 0.54 |
| 5 | 0.62 |
| 6 | 0.38 |

Table 8

| Malonyl CoA concentrations in Wistar rat brain | |
| --- | --- |
| Wistar rat individual No. | Malonyl CoA concentration (pmol/g wet wt.) |
| 1 | 21.7 |
| 2 | 28.3 |
| 3 | 70.3 |
| 4 | 77.2 |
| 5 | 44.2 |
| 6 | 57.3 |

EFFECT OF THE INVENTION

According to the assay method of the invention, CoA molecules in biological samples can be quantitatively assayed in a precise and reproducible manner by LC-MS.

Table 3  Results of reproducibility confirmation test using muscle samples

| Muscle QC sample | QC0 Endogenous level | QC1 0.25nmol/g wet wt. | QC2 2.5 nmol/g wet wt. | QC3 25 nmol/g wet wt. |
|---|---|---|---|---|
| mean (nmol/g wet wt.) | 5.31 | 0.239 | 2.61 | 26.4 |
| CV(%) | 1.7 | 9.1 | 5.6 | 2.6 |
| RE(%) | - | -4.3 | 4.5 | 5.7 |

Table 4  Results of reproducibility confirmation test using liver samples

| Liver QC sample | QC0 Endogenous level | QC1 0.25nmol/g wet wt. | QC2 2.5 nmol/g wet wt. | QC3 25 nmol/g wet wt. |
|---|---|---|---|---|
| mean (nmol/g wet wt.) | 0.50 | 0.272 | 2.67 | 23.6 |
| CV(%) | 8.2 | 4.8 | 5.0 | 3.0 |
| RE(%) | - | 9.1 | 6.8 | -5.6 |

Table 5 Results of reproducibility confirmation test using brain samples

| Brain QC sample | QC0 Endogenous level | QC1 125 pmol/g wet wt. | QC2 1250 pmol/g wet wt. | QC3 12500 pmol/g wet wt. |
|---|---|---|---|---|
| mean (pmol/g wet wt.) | 51.6 | 120.5 | 1336 | 13337 |
| CV (%) | 4.6 | 7.5 | 2.0 | 4.3 |
| RE (%) | - | -3.6 | 6.9 | 6.7 |

**Claims**

1. A method for assaying concentrations of Coenzyme A molecules in biological samples, the method being **characterized by** comprising a step of extraction from a biological sample using a strongly acidic solution, a step of solid phase extraction, a step of adding an internal standard substance, and a step of detection by LC-MS.

2. The method for assaying Coenzyme A molecules according to claim 1, **characterized in that** the step of extracting the Coenzyme A molecule from the biological sample is a step wherein a freeze-shattered biological sample is agitated in a perchloric acid solution and the supernatant is subjected to centrifugal separation.

3. The method for assaying Coenzyme A molecules according to claim 1 or 2, **characterized in that** the solid phase extraction step is a step wherein the supernatant obtained by extraction of the Coenzyme A with a strongly acidic solution is neutralized, and then applied to a reverse phase cartridge packed with silica gel containing an octadecylsilyl group or octylsilyl group, washed with an aqueous solvent, and eluted with an organic solvent.

4. The method for assaying Coenzyme A molecules according to claim 3, **characterized in that** the supernatant is applied after conditioning the reverse phase cartridge with acetonitrile and 1 M ammonium acetate solution.

5. The method for assaying Coenzyme A molecules according to claim 3, **characterized in that** the organic solvent is a mixture of acetonitrile and ammonium acetate.

6. The method for assaying Coenzyme A molecules according to claim 1, **characterized in that** the Coenzyme A molecule is a fatty acid Coenzyme A ester, and the internal standard substance is a structural analog of the Coenzyme A molecule.

7. The method for assaying Coenzyme A molecules according to claim 6, **characterized in that** the fatty acid Coenzyme A ester is a Coenzyme A ester of a short chain fatty acid with 2-8 carbons in the main carbon chain, and the structural analog has a difference of no more than 3 carbons with the Coenzyme A molecule and has at least 3 of the hydrogens of the main chain substituted with deuterium, or has at least 3 of the carbons of the main carbon chain substituted with $^{13}$C.

8. The method for assaying Coenzyme A molecules according to claim 7, **characterized in that** the Coenzyme A molecule is malonyl CoA and the structural analog is acetyl CoA-$d_3$, methylmalonyl CoA-$d_3$, methylmalonyl CoA-$d_4$, propionyl CoA-$d_3$, propionyl CoA-$d_5$ or malonyl CoA-$^{13}$C$_3$.

Fig. 1

Malonyl CoA

Acetyl CoA-d3

Fig. 2

Malonyl CoA

Acetyl CoA-d3

Fig. 3

## INTERNATIONAL SEARCH REPORT

| | International application No. |
| --- | --- |
| | PCT/JP03/03521 |

### A. CLASSIFICATION OF SUBJECT MATTER
Int.Cl$^7$ G01N30/88, G01N30/72, G01N30/04, G01N27/62

According to International Patent Classification (IPC) or to both national classification and IPC

### B. FIELDS SEARCHED

Minimum documentation searched (classification system followed by classification symbols)
Int.Cl$^7$ G01N30/88, G01N30/72, G01N30/04, G01N27/62

Documentation searched other than minimum documentation to the extent that such documents are included in the fields searched
| | | | |
| --- | --- | --- | --- |
| Jitsuyo Shinan Koho | 1922–1996 | Toroku Jitsuyo Shinan Koho | 1994–2003 |
| Kokai Jitsuyo Shinan Koho | 1971–2003 | Jitsuyo Shinan Toroku Koho | 1996–2003 |

Electronic data base consulted during the international search (name of data base and, where practicable, search terms used)
CA(STN)

### C. DOCUMENTS CONSIDERED TO BE RELEVANT

| Category* | Citation of document, with indication, where appropriate, of the relevant passages | Relevant to claim No. |
| --- | --- | --- |
| Y/A | BUCHHOLZ et al., "Quantitation of Intracellular Metabolites in Escherichia coli K12 Using Liquid Chromatographic-Electrospray Ionization Tandem Mass Spectrometric Techniques", Analytical Biochemistry, Vol.295, (2001), pages 129 to 137 | 1,2,6-8/3-5 |
| Y/A | WO 00/11208 A (UNIVERSITY OF WASHINGTON), 02 March, 2000 (02.03.00), Page 8, line 19 to page 9, line 17 & EP 2205517 A & JP 2002-523058 A | 1,2,6-8/3-5 |

☐ Further documents are listed in the continuation of Box C.  ☐ See patent family annex.

| | |
| --- | --- |
| \* Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 28 May, 2003 (28.05.03) | 17 June, 2003 (17.06.03) |

| Name and mailing address of the ISA/ | Authorized officer |
| --- | --- |
| Japanese Patent Office | |
| Facsimile No. | Telephone No. |

Form PCT/ISA/210 (second sheet) (July 1998)